# EUROPÄISCHE PATENTSCHRIFT

(11) **EP 1 218 339 B1**
(45) Veröffentlichungstag und Bekanntmachung des Hinweises auf die Patenterteilung: **01.10.2003**
(21) Anmeldenummer: 00992195.8
(22) Anmeldetag: 05.10.2000
(51) Int. Cl.: C07C 259/14, C07D 213/54, C07D 333/24, A01N 37/52, A01N 43/10, A01N 43/40

(54) **BENZYLAMINODOXIM-DERIVATE, ZWISCHENPRODUKTE UND VERFAHREN ZU DEREN HERSTELLUNG UND DEREN VERWENDUNG ALS FUNGIZIDE**
BENZYL AMIDOXIME DERIVATIVES, INTERMEDIATE PRODUCTS AND METHOD FOR THEIR PRODUCTION AND USE AS FUNGICIDES
DERIVES DE BENZYLAMIDOXIME, PRODUITS INTERMEDIAIRES ET PROCEDE SERVANT A LA PREPARATION DESDITS DERIVES, AINSI QUE LEUR UTILISATION COMME FONGICIDES

(30) Priorität: 06.10.1999 DE 19948266
(43) Veröffentlichungstag der Anmeldung: 03.07.2002
(73) Patentinhaber: BASF AKTIENGESELLSCHAFT, 67056 Ludwigshafen (DE)
(72) Erfinder: RHEINHEIMER, Joachim, 67063 Ludwigshafen (DE); EICKEN, Karl, 67157 Wachenheim (DE); ROSE, Ingo, 68159 Mannheim (DE); GROTE, Thomas, 67157 Wachenheim (DE); AMMERMANN, Eberhard, 64646 Heppenheim (DE); SPEAKMAN, John-Bryan, 67273 Bobenheim (DE); STRATHMANN, Siegfried, 67117 Limburgerhof (DE); LORENZ, Gisela, 67434 Neustadt (DE)
(86) Internationale Anmeldenummer: EP0009744
(87) Internationale Veröffentlichungsnummer: WO01025187

(56) Entgegenhaltungen:
- BE-A- 826 325
- DE-C- 540 409
- GB-A- 876 079
- PATENT ABSTRACTS OF JAPAN vol. 1998, no. 69, 31. Juli 1998 (1998-07-31) & JP 10 095771 A (NIPPON SODA CO LTD), 14. April 1998 (1998-04-14) in der Anmeldung erwähnt -& JP 10 095771 A 14. April 1998 (1998-04-14)
- CHEMICAL ABSTRACTS, vol. 62, no. 10, 10. Mai 1965 (1965-05-10) Columbus, Ohio, US; abstract no. 11732e, A. A. AROYAN ET AL.: "Synthesis of some amines amidoximes, and derivatives of guanidine" Spalte 11732; XP002164076 & IZV. AKAD. NAUK ARM. SSR, KHIM. NAUKI, Bd. 17, Nr. 5, 1964, Seiten 543-548,
- SEIGO SUZUE ET AL.: "Synthetic antimicrobials" CHEMICAL AND PHARMACEUTICAL BULLETIN., Bd. 21, 1973, Seiten 2146 -2160, XP000926253 PHARMACEUTICAL SOCIETY OF JAPAN. TOKYO., JP ISSN: 0009-2363
- BOUALEM OUSSAID ET AL.: "Improved synthesis of oxadiazoles under microwave irradation" SYNTHETIC COMMUNICATIONS., Bd. 25, Nr. 10, 1995, Seiten 1451-1459, XP000926265 MARCEL DEKKER, INC., BASEL., CH ISSN: 0039-7911

## Beschreibung

Die vorliegende Erfindung betrifft neue Benzylamidoxim-Derivate, Verfahren und Zwischenprodukte zu deren Herstellung und deren Verwendung als Fungizide.

In der japanischen Offenlegungsschrift JP 10-95771 sind unter anderem fungizide Benzylamidoxime beschrieben, die jedoch hinsichtlich ihrer fungiziden Wirkung und biologischen Eigenschaften nicht in vollem Umfang befriedigen können.
Aufgabe der vorliegenden Erfindung war es daher, neue Benzamidoxim-Derivate mit verbesserten biologischen Eigenschaften und erhöhter Wirkung, insbesondere auch bei niedrigen Aufwandmengen, zur Verfügung zu stellen.

Gegenstand der vorliegenden Anmeldung sind daher Benzalamidoxim-Derivate der Formel I 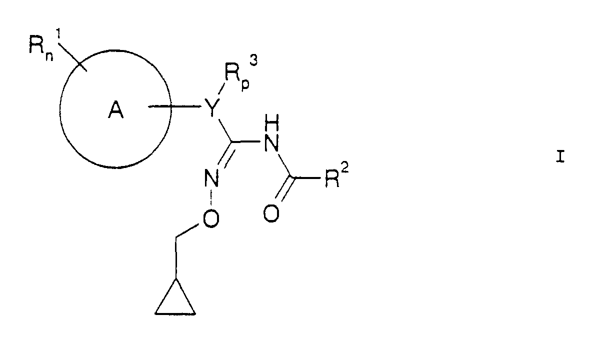 wobei die Reste folgende Bedeutung haben:
- A: ein Aryl oder Hetarylrest aus der Gruppe Phenyl, Pyridyl oder Thienyl;
- Y: eine geradkettige oder verzweigte C₁-C₄-Alkylengruppe, wobei ein Kohlenstoffatom durch ein Sauerstoff-, Stickstoff- oder Schwefelatom oder durch eine Cyclopropylgruppe ersetzt sein kann;
- Rₙ¹: ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
- R²: Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazolyl-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann,
- Rₚ³: ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy, C₁-C₆-Alkylcarbonyl;
- n: 0, 1, 2, 3, 4 oder 5;
- p: je nach Anzahl der freien Valenzen 0, 1, 2, 3, 4
sowie deren umweltverträgliche und landwirtschaftlich einsetzbare Salze.

Die Zahlen n und p geben die Anzahl der Substituenten R¹ bzw. R³ an. Wenn n = 0 ist, bedeutet R¹ Wasserstoff, Wenn p = 0 ist bedeutet R³ Wasserstoff.

Bei der Definition der in der Formel I angegebenen Reste stehen die genannten Begriffe als Sammelbegriff für eine Gruppe von Verbindungen.

Die Bedeutung Halogen steht jeweils für Fluor, Brom, Chlor oder Iod, insbesondere für Fluor oder Chlor.

Ferner stehen beispielsweise:
- C₁-C₆-Alkyl für: Methyl, Ethyl, n-Propyl, 1-Methylethyl, n-Butyl, 1-Methylpropyl, 2-Methylpropyl oder 1,1-Dimethylethyl, insbesondere für C₁-C₄-Alkyl, sowie für Methyl oder Ethyl;
- C₁-C₆-Halogenalkyl für: einen C₁-C₆-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere für Trifluormethyl;
- C₁-C₄-Alkylen für: eine geradkettige oder verzweigte Kohlenstoffkette, wie z.B. -CH₂-, -CH₂-CH₂-, -CH(CH₃)-, -CH₂-CH(CH₃)-, CH(CH₃)-CH₂-, CH₂-CH₂-CH₂-, -CH₂-CH(CH₃)-CH₂-;
- C₁-C₄-Alkylen, wobei ein Kohlenstoffatom durch ein Sauerstoff, Schwefel- oder Stickstoffatom ersetzt sein kann, für: eine C₁-C₄-Alkylen wie zuvor genannt, wobei jedes beliebige Kohlenstoffatom durch ein Heteroatom X (X=O, S, NH) ersetzt sein kann, wie z.B. -X-CH₂-, -CH₂-X-, -X-CH₂-CH₂-, -CH(CH₃)-X-, -X-CH₂-CH(CH₃)-, CH(CH₃)-CH₂-X-, -X-CH₂-CH₂-CH₂-, -CH₂-CH(CH₃)-CH₂-X-;
- C₁-C₄-Alkylen, wobei ein Kohlenstoffatom durch eine gegebenenfalls durch R³ₚ substituierte Cyclopropylgruppe (cPr) ersetzt sein kann, für: eine C₁-C₄-Alkylen wie zuvor genannt, wobei jedes beliebige Kohlenstoffatom durch ein Heteroatom X (X=O, S, NH) ersetzt sein kann, wie z.B. -cPr-, -cPr-CH₂-, -CH₂-cPr-, -cPr-CH₂-CH₂-, -CH(CH₃)-CPr-, -cPr-CH₂-CH(CH₃)-, CH(CH₃)-CH₂-cPr-, -cPr-CH₂-CH₂-CH₂-, -CH₂-CH(CH₃)-CH₂-cPr-;
- C₁-C₆-Alkoxy für: Methoxy, Ethoxy, n-Propoxy, 1-Methylethoxy, n-Butoxy, 1-Methylpropoxy, 2-Methylpropoxy oder 1,1-Dimethylethoxy, insbesondere für C₁-C₄-Alkoxy, sowie für Methoxy oder Ethoxy;
- C₁-C₆-Halogenalkoxy für: einen C₁-C₄-Alkoxyrest wie vorstehend genannt, der partiell oder vollständig durch Fluor, Chlor, Brom und/oder Iod substituiert ist, also z.B. Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für Difluormethoxy;
- Phenyl-C₁-C₆-alkyl für: z.B. Benzyl, 1-Phenylethyl, 2-Phenylethyl, 1-Phenylprop-1-yl, 2-Phenylprop-1-yl, 3-Phenylprop-1-yl, 1-(Phenylmethyl)-eth-1-yl, 1-(Phenylmethyl)-1-(methyl)-eth-1-yl oder 1-(Phenylmethyl)-prop-1-yl, insbesondere für Benzyl oder 2-Phenylethyl;
- Thienyl-C₁-C₄-alkyl für: z.B. 2-Thienylmethyl, 3-Thienylmethyl, 2-Thienylethyl, 2-Thienylprop-1-yl oder 3-Thienylprop-1-yl;
- Pyrazol-C₁-C₄-alkyl für: z.B. 1-Pyrazolyl-methyl, 2-Pyrazolylmethyl, 3-Pyrazolylmethyl, 2-Pyrazolylylethyl, 2-Pyrazolylylprop-1-yl oder 3-Pyrazolylprop-1-yl;
- Heteroaryl: ein aromatischer 5- oder 6-gliedriger heterocyclischer Ring, der ein bis vier gleiche oder verschiedene Heteroatome, ausgewählt aus folgender Gruppe: Sauerstoff, Schwefel oder Stickstoff, enthält, und über ein Kohlenstoffatom oder ein Heteroatom an die Gruppe Y gebunden sein kann; wie z.B. Pyridyl, Pyrrolyl, Pyrimidinyl, Imidazolyl, Pyrazolyl, Thienyl, Oxazinyl, Furanyl, Oxazolyl, Imidoxazolyl;
- Aryl: ein aromatischer carbocyclischer, mono- oder bicyclischer Ring mit 6 - 14 Kohlenstoffatomen, wie z.B. Phenyl, Naphthyl; insbesondere Phenyl. Verbindungen der Formel I, in denen A eine Phenylgruppe und n die Zahlen 1, 2 oder 3 darstellt, haben sich als in der Regel besonders wirksam erwiesen. R¹ bedeutet hierbei bevorzugt Fluor, Chlor, Methyl, Methoxy oder Trifluormethyl.

Für den Fall, daß A eine Phenylgruppe bedeutet, kommen als Substituenten R¹ₙ bevorzugt folgende Bedeutungen in Frage: 2,6-Dichlor; 2-Chlor-6-fluor; 2,6-Difluor; 2-Chlor-5,6-difluor; 2-Chlor-6-trifluormethyl; 2-Fluor-6-trifluormethyl; 2-Brom-6-trifluormethyl; 2-Iod-6-trifluormethyl; 2,6-Dibrom; 2-Brom-6-fluor; 2-Brom-6-chlor; 2-Chlor-6-trifluormethoxy; 2-Fluor-6-trifluormethoxy; 2-Chlor-6-difluormethoxy; 2-Difluormethoxy-6-fluor; 2,3-Dichlor-6-difluormethoxy; 2,3-Difluor-6-difluormethoxy; 2,6-Bis(difluormethoxy); 2,6-Bis(trfluormethoxy); 2,6-Bis(trfluormethyl); 2-Brom; 2-Chlor; 2-Fluor; 3-Brom; 3-Chlor; 3-Fluor; 4-Brom; 4-Chlor; 4-Fluor; 4-Methoxy; 2-Chlor-6-methylthio; 2,3-Difluor-6-methylthio; 2,4-Dichlor; 3,5-Dichlor; 2,3,6-Trichlor; 2,3,6-Trifluor; 2,3,4,5,6-Pentafluor; 2-Fluor-6-methyl; 2-Chlor-6-methyl.

Die Gruppe R² bedeutet vorzugsweise Phenylmethyl; (4-Chlorphenyl)methyl; (4-Fluorphenyl)methyl; (4-Methylphenyl)methyl; (3-Methylphenyl)methyl; (4-Trifluormethylphenyl)methyl; (4-Methoxyphenyl)methyl; 2-Thienyl)methyl.

Y bedeutet insbesondere eine geradkettige oder verzweigte C₁-C₃-Alkylenkette, wobei ein Kohlenstoffatom durch ein Sauerstoff- oder Schwefelatom bzw. eine Iminogruppe (-NH-) oder Alkyliminogruppe (-N(Alkyl)-) ersetzt sein kann.

Bevorzugt sind Verbindungen der Formel 1, wobei die Reste folgende Bedeutungen haben:
- A: ein Aryl oder Hetarylrest aus der Gruppe Phenyl, Pyridyl oder Thienyl;
- Y: ein Kohlenstoffatom;
- Rₙ¹: ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
- R²: Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazolyl-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann,
- Rₚ³: Wasserstoff oder C₁-C₄-Alkyl;
- n: 0-5;
- p: 0-2.

Besonders bevorzugt sind Verbindungen der Formel 1, wobei die Reste folgende Bedeutungen haben:
- A: Phenyl;
- Y: ein Kohlenstoffatom;
- Rₙ¹: ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
- R²: Phenyl-Methyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann;
- Rₚ³: Wasserstoff oder Methyl;
- n: 0-5;
- p: 0-1.

Verbindungen der Formel I, in denen R¹ und R² die in der nachstehenden Tabelle 1 aufgeführten Bedeutungen haben, sind insbesondere bevorzugt.

**Tabelle 1:**

| **Nr. A** | **R**^{**1**}_{**n**} | **R**^{**2**} | **Y-R**^{**3**}_{**p**} |
|---|---|---|---|
| 1) Phenyl | 2,6-Dichlor | Phenylmethyl | -CH₂- |
| 2) Phenyl | 2-Chlor-6-fluor | Phenylmethyl | -CH₂- |
| 3) Phenyl | 2,6-Difluor | Phenylmethyl | -CH₂- |
| 4) Phenyl | 2-Chlor-5,6-difluor | Phenylmethyl | -CH₂- |
| 5) Phenyl | | Phenylmethyl | -CH₂- |
| 6) Phenyl | 2-Chlor-6-trifluormethyl | Phenylmethyl | -CH₂- |
| 7) Phenyl | 2-Fluor-6-trifluormethyl | Phenylmethyl | -CH₂- |
| 8) Phenyl | 2-Brom-6-trifluormethyl | Phenylmethyl | -CH₂- |
| 9) Phenyl | 2-lod-6-trifluormethyl | Phenylmethyl | -CH₂- |
| 10) Phenyl | 2,6-Dibrom | Phenylmethyl | -CH₂- |
| 11) Phenyl | 2-Brom-6-fluor | Phenylmethyl | -CH₂- |
| 12) Phenyl | 2-Brom-6-chlor | Phenylmethyl | -CH₂- |
| 13) Phenyl | 2-Chlor-6-trifluormethoxy | Phenylmethyl | -CH₂- |
| 14) Phenyl | 2-Fluor-6-trifluormethoxy | Phenylmethyl | -CH₂- |
| 15) Phenyl | 2-Chlor-6-difluormethoxy | Phenylmethyl | -CH₂- |
| 16) Phenyl | 2-Difluormethoxy-6-fluor | Phenylmethyl | -CH₂- |
| 17) Phenyl | 2,3-Dichlor-6-difluormethoxy | Phenylmethyl | -CH₂- |
| 18) Phenyl | 2,3-Difluor-6-difluormethoxy | Phenylmethyl | -CH₂- |
| 19) Phenyl | 26-Bis(difluormethoxy) | Phenylmethyl | -CH₂- |
| 20) Phenyl | 2,6-Bis(trfluormethoxy) | Phenylmethyl | -CH₂- |
| 21) Phenyl | 2,6-Bis(trfluormethyl) | Phenylmethyl | -CH₂- |
| 22) Phenyl | 2-Brom | Phenylmethyl | -CH₂- |
| 23) Phenyl | 2-Chlor | Phenylmethyl | -CH₂- |
| 24) Phenyl | 2-Fluor | Phenylmethyl | -CH₂- |
| 25) Phenyl | 3-Brom | Phenylmethyl | -CH₂- |
| 26) Phenyl | 3-Chlor | Phenylmethyl | -CH₂- |
| 27) Phenyl | 3-Fluor | Phenylmethyl | -CH₂- |
| 28) Phenyl | 4-Brom | Phenylmethyl | -CH₂- |
| 29) Phenyl | 4-Chlor | Phenylmethyl | -CH₂- |
| 30) Phenyl | 4-Fluor | Phenylmethyl | -CH₂- |
| 31) Phenyl | 4-Methoxy | Phenylmethyl | -CH₂- |
| 32) Phenyl | 2-Chlor-6-methylthio | Phenylmethyl | -CH₂- |
| 33) Phenyl | 2,3-Difluor-6-methylthio | Phenylmethyl | -CH₂- |
| 34) Phenyl | 2,4-Dichlor | Phenylmethyl | -CH₂- |
| 35) Phenyl | 3,5-Dichlor | Phenylmethyl | -CH₂- |
| 36) Phenyl | 2,3,6-Trichlor | Phenylmethyl | -CH2- |
| 37) Phenyl | 2,3,6-Trifluor | Phenylmethyl | -CH₂- |
| 38) Phenyl | 2,3,4,5,6-Pentafluor | Phenylmethyl | -CH₂- |
| 39) Phenyl | 2-Fluor-6-methyl | Phenylmethyl | -CH₂- |
| 40) Phenyl | 2-Chlor-6-methyl | Phenylmethyl | -CH₂- |
| 41) Phenyl | 2,6-Dichlor | Phenylmethyl | -CH₂ CH₂- |
| 42) Phenyl | 2-Chlor-6-fluor | Phenylmethyl | -CH₂ CH₂- |
| 43) Phenyl | 2,6-Difluor | Phenylmethyl | -CH₂ CH₂- |
| 44) Phenyl | 2-Chlor-5,6-difluor | Phenylmethyl | -CH₂ CH₂- |
| 45) Phenyl | 2-Chlor-6-trifluormethyl | Phenylmethyl | -CH₂ CH₂- |
| 46) Phenyl | 2-Fluor-6-trifluormethyl | Phenylmethyl | -CH₂ CH₂- |
| 47) Phenyl | 2-Brom-6-trifluormethyl | Phenylmethyl | -CH₂ CH₂- |
| 48) Phenyl | 2-lod-6-trifluormethyl | Phenylmethyl | -CH₂ CH₂- |
| 49) Phenyl | 2,6-Dibrom | Phenylmethyl | -CH₂ CH₂- |
| 50) Phenyl | 2-Brom-6-fluor | Phenylmethyl | -CH₂ CH₂- |
| 51) Phenyl | 12-Brom-6-chlor | Phenylmethyl | -CH₂ CH₂- |
| 52) Phenyl | 2-Chlor-6-trifluormethoxy | Phenylmethyl | -CH₂ CH₂- |
| 53) Phenyl | 2-Fluor-6-trifluormethoxy | Phenylmethyl | -CH₂ CH₂- |
| 54) Phenyl | 2-Chlor-6-difluormethoxy | Phenylmethyl | -CH₂ CH₂- |
| 55) Phenyl | 2-Difluormethoxy-6-fluor | Phenylmethyl | -CH₂ CH₂- |
| 56) Phenyl | 2,3-Dichlor-6-difluormethoxy | Phenylmethyl | -CH₂ CH₂- |
| 57) Phenyl | 2,3-Difluor-6-difluormethoxy | Phenylmethyl | -CH₂ CH₂- |
| 58) Phenyl | 2,6-Bis(difluormethoxy) | Phenylmethyl | -CH₂ CH₂- |
| 59) Phenyl | 2,6-Bis(trfluormethoxy) | Phenylmethyl | -CH₂ CH₂- |
| 60) Phenyl | 2,6-Bis(trfluormethyl) | Phenylmethyl | -CH₂ CH₂- |
| 61) Phenyl | 2-Brom | Phenylmethyl | -CH₂ CH₂- |
| 62) Phenyl | 2-Chlor | Phenylmethyl | -CH₂ CH₂- |
| 63) Phenyl | 2-Fluor | Phenylmethyl | -CH₂ CH₂- |
| 64) Phenyl | 3-Brom | Phenylmethyl | -CH₂ CH₂- |
| 65) Phenyl | 3-Chlor | Phenylmethyl | -CH₂ CH₂- |
| 66) Phenyl | 3-Fluor | Phenylmethyl | -CH₂ CH₂- |
| 67) Phenyl | 4-Brom | Phenylmethyl | -CH₂ CH₂- |
| 68) Phenyl | 4-Chlor | Phenylmethyl | -CH₂ CH₂- |
| 69) Phenyl | 4-Fluor | Phenylmethyl | -CH₂ CH₂- |
| 70) Phenyl | 4-Methoxy | Phenylmethyl | -CH₂ CH₂- |
| 71) Phenyl | 2-Chlor-6-methylthio | Phenylmethyl | -CH₂ CH₂- |
| 72) Phenyl | 2,3-Difluor-6-methylthio | Phenylmethyl | -CH₂ CH₂- |
| 73) Phenyl | 2,4-Dichlor | Phenylmethyl | -CH₂ CH₂- |
| 74) Phenyl | 3,5-Dichlor | Phenylmethyl | -CH₂ CH₂- |
| 75) Phenyl | 2,3,6-Trichlor | Phenylmethyl | -CH₂ CH₂- |
| 76) Phenyl | 2,3,6-Trifluor | Phenylmethyl | -CH₂ CH₂- |
| 77) Phenyl | 2,3,4,5,6-Pentafluor | Phenylmethyl | -CH₂ CH₂- |
| 78) Phenyl | 2-Fluor-6-methyl | Phenylmethyl | -CH₂ CH₂- |
| 79) Phenyl | 2-Chlor-6-methyl | Phenylmethyl | -CH₂ CH₂- |
| 80) Phenyl | 2,6-Dichlor | (4-Chlorphenyl)methyl | -CH₂- |
| 81) Phenyl | 2-Chlor-6-fluor | (4-Chlorphenyl)methyl | -CH₂- |
| 82) Phenyl | 2,6-Difluor | (4-Chlorphenyl)methyl | -CH₂- |
| 83) Phenyl | 2-Chlor-5,6-difluor | (4-Chlorphenyl)methyl | -CH₂- |
| 84) Phenyl | 2-Chlor-6-trifluormethyl | (4-Chlorphenyl)methyl | -CH₂- |
| 85) Phenyl | 2-Fluor-6-trifluormethyl | (4-Chlorphenyl)methyl | -CH₂- |
| 86) Phenyl | 2-Brom-6-trifluormethyl | (4-Chlorphenyl)methyl | -CH₂- |
| 87) Phenyl | 2-lod-6-trifluormethyl | (4-Chlorphenyl)methyl | -CH₂- |
| 88) Phenyl | 2,6-Dibrom | (4-Chlorphenyl)methyl | -CH₂- |
| 89) Phenyl | 2-Brom-6-fluor | (4-Chlorphenyl)methyl | -CH₂- |
| 90) Phenyl | 2-Brom-6-chlor | (4-Chlorphenyl)methyl | -CH₂- |
| 91) Phenyl | 2-Chlor-6-trifluormethoxy | (4-Chlorphenyl)methyl | -CH₂- |
| 92) Phenyl | 2-Fluor-6-trifluormethoxy | (4-Chlorphenyl)methyl | -CH₂- |
| 93) Phenyl | 2-Chlor-6-difluormethoxy | (4-Chlorphenyl)methyl | -CH₂- |
| 94) Phenyl | 2-Difluormethoxy-6-fluor | (4-Chlorphenyl)methyl | -CH₂- |
| 95) Phenyl | 2,3-Dichior-6-difiuormethoxy | (4-Chlorphenyl)methyl | -CH₂- |
| 96) Phenyl | 2,3-Difluor-6-difluormethoxy | (4-Chlorphenyl)methyl | -CH₂- |
| 97) Phenyl | 2,6-Bis(difluormethoxy) | (4-Chlorphenyl)methyl | -CH₂- |
| 98) Phenyl | 2,6-Bis(trfluormethoxy) | (4-Chlorphenyl)methyl | -CH₂- |
| 99) Phenyl | 2,6-Bis(trfluormethyl) | (4-Chlorphenyl)methyl | -CH₂- |
| 100) Phenyl | 2-Brom | (4-Chlorphenyl)methyl | -CH₂- |
| 101) Phenyl | 2-Chlor | (4-Chlorphenyl)methyl | -CH₂- |
| 102) Phenyl | 2-Fluor | (4-Chlorphenyl)methyl | -CH₂- |
| 103) Phenyl | 3-Brom | (4-Chlorphenyl)methyl | -CH₂- |
| 104) Phenyl | 3-Chlor | (4-Chlorphenyl)methyl | -CH₂- |
| 105) Phenyl | 3-Fluor | (4-Chlorphenyl)methyl | -CH₂- |
| 106) Phenyl | 4-Brom | (4-Chlorphenyl)methyl | -CH₂- |
| 107) Phenyl | 4-Chlor | (4-Chlorphenyl)methyl | -CH₂- |
| 108) Phenyl | 4-Fluor | (4-Chlorphenyl)methyl | -CH₂- |
| 109) Phenyl | 4-Methoxy | (4-Chlorphenyl)methyl | -CH₂- |
| 110) Phenyl | 2-Chlor-6-methylthio | (4-Chlorphenyl)methyl | -CH₂- |
| 111) Phenyl | 2,3-Difluor-6-methylthio | (4-Chlorphenyl)methyl | -CH₂- |
| 112) Phenyl | 2,4-Dichlor | (4-Chlorphenyl)methyl | -CH₂- |
| 113) Phenyl | 3,5-Dichlor | (4-Chlorphenyl)methyl | -CH₂- |
| 114) Phenyl | 2,3,6-Trichlor | (4-Chlorphenyl)methyl | -CH₂- |
| 115) Phenyl | 2,3,6-Trifluor | (4-Chlorphenyl)methyl | -CH₂- |
| 116) Phenyl | 2,3,4,5,6-Pentafluor | (4-Chlorphenyl)methyl | -CH₂- |
| 117) Phenyl | 2-Fluor-6-methyl | (4-Chlorphenyl)methyl | -CH₂- |
| 118) Phenyl | 2-Chlor-6-methyl | (4-Chlorphenyl)methyl | -CH₂- |
| 119) Phenyl | 2,6-Dichlor | (4-Fluorphenyl)methyl | -CH₂- |
| 120) Phenyl | 2-Chlor-6-fluor | (4-Fluorphenyl)methyl | -CH₂- |
| 121) Phenyl | 2,6-Difluor | (4-Fluorphenyl)methyl | -CH₂- |
| 122) Phenyl | 2-Chlor-5,6-difluor | (4-Fluorphenyl)methyl | -CH₂- |
| 123) Phenyl | 2-Chlor-6-trifluormethyl | (4-Fluorphenyl)methyl | -CH₂- |
| 124) Phenyl | 2-Fluor-6-trifluormethyl | (4-Fluorphenyl)methyl | -CH₂- |
| 125) Phenyl | 2-Brom-6-trifluormethyl | (4-Fluorphenyl)methyl | -CH₂- |
| 126) Phenyl | 2-Iod-6-trifluormethyl | (4-Fluorphenyl)methyl | -CH₂- |
| 127) Phenyl | 2,6-Dibrom | (4-Fluorphenyl)methyl | -CH₂- |
| 128) Phenyl | 2-Brom-6-fluor | (4-Fluorphenyl)methyl | -CH₂- |
| 129) Phenyl | 2-Brom-6-chlor | (4-Fluorphenyl)methyl | -CH₂- |
| 130) Phenyl | 2-Chlor-6-trifluormethoxy | (4-Fluorphenyl)methyl | -CH₂- |
| 131) Phenyl | 2-Fluor-6-trifluormethoxy | (4-Fluorphenyl)methyl | -CH₂- |
| 132) Phenyl | 2-Chlor-6-difluormethoxy | (4-Fluorphenyl)methyl | -CH₂- |
| 133) Phenyl | 2-Difluormethoxy-6-fluor | (4-Fluorphenyl)methyl | -CH₂- |
| 134) Phenyl | 2,3-Dichlor-6-difluormethoxy | (4-Fluorphenyl)methyl | -CH₂- |
| 135) Phenyl | 2,3-Difluor-6-difluormethoxy | (4-Fluorphenyl)methyl | -CH₂- |
| 136) Phenyl | 2,6-Bis(difluormethoxy) | (4-Fluorphenyl)methyl | -CH₂- |
| 137) Phenyl | 2,6-Bis(trfluormethoxy) | (4-Fluorphenyl)methyl | -CH₂- |
| 138) Phenyl | 2,6-Bis(trfluormethyl) | (4-Fluorphenyl)methyl | -CH₂- |
| 139) Phenyl | 2-Brom | (4-Fluorphenyl)methyl | -CH₂- |
| 140) Phenyl | 2-Chlor | (4-Fluorphenyl)methyl | -CH₂- |
| 141) Phenyl | 2-Fluor | (4-Fluorphenyl)methyl | -CH₂- |
| 142) Phenyl | 3-Brom | (4-Fluorphenyl)methyl | -CH₂- |
| 143) Phenyl | 3-Chlor | (4-Fluorphenyl)methyl | -CH₂- |
| 144) Phenyl | 3-Fluor | (4-Fluorphenyl)methyl | -CH₂- |
| 145) Phenyl | 4-Brom | (4-Fluorphenyl)methyl | -CH₂- |
| 146) Phenyl | 4-Chlor | (4-Fluorphenyl)methyl | -CH₂- |
| 147) Phenyl | 4-Fluor | (4-Fluorphenyl)methyl | -CH₂- |
| 148) Phenyl | 4-Methoxy | (4-Fluorphenyl)methyl | -CH₂- |
| 149) Phenyl | 2-Chlor-6-methylthio | (4-Fluorphenyl)methyl | -CH₂- |
| 150) Phenyl | 2,3-Difluor-6-methylthio | (4-Fluorphenyl)methyl | -CH₂- |
| 151) Phenyl | 2,4-Dichlor | (4-Fluorphenyl)methyl | -CH₂- |
| 152) Phenyl | 3,5-Dichlor | (4-Fluorphenyl)methyl | -CH₂- |
| 153) Phenyl | 2,3,6-Trichlor | (4-Fluorphenyl)methyl | -CH₂- |
| 154) Phenyl | 2,3,6-Trifluor | (4-Fluorphenyl)methyl | -CH₂- |
| 155) Phenyl | 2,3,4,5,6-Pentafluor | (4-Fluorphenyl)methyl | -CH₂- |
| 156) Phenyl | 2-Fluor-6-methyl | (4-Fluorphenyl)methyl | -CH₂- |
| 157) Phenyl | 2-Chlor-6-methyl | (4-Fluorphenyl)methyl | -CH₂- |
| 158) Phenyl | 2,6-Dichlor | (4-Methylphenyl)methyl | -CH₂- |
| 159) Phenyl | 2-Chlor-6-fluor | (4-Methylphenyl)methyl | -CH₂- |
| 160) Phenyl | 2,6-Difluor | (4-Methylphenyl)methyl | -CH₂- |
| 161) Phenyl | 2-Chlor-5.6-difluor | (4-Methylphenyl)methyl | -CH₂- |
| 162) Phenyl | 2-Chlor-6-trifluormethyl | (4-Methylphenyl)methyl | -CH₂- |
| 163) Phenyl | 2-Fiuor-6-trifluormethyl | (4-Methylphenyl)methyl | -CH₂- |
| 164) Phenyl | 2-Brom-6-trifluormethyl | (4-Methylphenyl)methyl | -CH₂- |
| 165) Phenyl | 2-lod-6-trifluormethyl | (4-Methylpnenyl)methyl | -CH₂- |
| 166) Phenyl | 2,6-Dibrom | (4-Methylphenyl)methyl | -CH₂- |
| 167) Phenyl | 2-Brom-6-fluor | (4-Methylphenyl)methyl | -CH₂- |
| 168) Phenyl | 2-Brom-6-chlor | (4-Methylphenyl)methyl | -CH₂- |
| 169) Phenyl | 2-Chlor-6-trifluormethoxy | (4-Methylphenyl)methyl | -CH₂- |
| 170) Phenyl | 2-Fluor-6-trifluormethoxy | (4-Methylphenyl)methyl | -CH₂- |
| 171) Phenyl | 2-Chlor-6-difluormethoxy | (4-Methylphenyl)methyl | -CH₂- |
| 172) Phenyl | 2-Difluormethoxy-6-fluor | (4-Methylphenyl)methyl | -CH₂- |
| 173) Phenyl | 2,3-Dichlor-6-difluormethoxy | (4-Methylphenyl)methyl | -CH₂- |
| 174) Phenyl | 2,3-Difluor-6-difluormethoxy | (4-Methylphenyl)methyl | -CH₂- |
| 175) Phenyl | 2,6-Bis(difluormethoxy) | (4-Methylphenyl)methyl | -CH₂- |
| 176) Phenyl | 2,6-Bis(trfluormethoxy) | (4-Methylphenyl)methyl | -CH₂- |
| 177) Phenyl | 2,6-Bis(trfluormethyl) | (4-Methylphenyl)methyl | -CH₂- |
| 178) Phenyl | 2-Brom | (4-Methylphenyl)methyl | -CH₂- |
| 179) Phenyl | 2-Chlor | (4-Methylphenyl)methyl | -CH₂- |
| 180) Phenyl | 2-Fluor | (4-Methylphenyl)methyl | -CH₂- |
| 181) Phenyl | 3-Brom | (4-Methylphenyl)methyl | -CH₂- |
| 182) Phenyl | 3-Chlor | (4-Methylphenyl)methyl | -CH₂- |
| 183) Phenyl | 3-Fluor | (4-Methylphenyl)methyl | -CH₂- |
| 184) Phenyl | 4-Brom | (4-Methylphenyl)methyl | -CH₂- |
| 185) Phenyl | 4-Chlor | (4-Methylphenyl)methyl | -CH₂- |
| 186) Phenyl | 4-Fluor | (4-Methylphenyl)methyl | -CH₂- |
| 187) Phenyl | 4-Methoxy | (4-Methylphenyl)methyl | -CH₂- |
| 188) Phenyl | 2-Chlor-6-methylthio | (4-Methylphenyl)methyl | -CH₂- |
| 189) Phenyl | 2,3-Difluor-6-methylthio | (4-Methylphenyl)methyl | -CH₂- |
| 190) Phenyl | 2,4-Dichlor | (4-Methylphenyl)methyl | -CH₂- |
| 191) Phenyl | 3,5-Dichlor | (4-Methylphenyl)methyl | -CH₂- |
| 192) Phenyl | 2,3,6-Trichlor | (4-Methylphenyl)methyl | -CH₂- |
| 193) Phenyl | 2,3,6-Trifluor | (4-Methylphenyl)methyl | -CH₂- |
| 194) Phenyl | 2,3,4,5,6-Pentafluor | (4-Methylphenyl)methyl | -CH₂- |
| 195) Phenyl | 2-Fluor-6-methyl | (4-Methylphenyl)methyl | -CH₂- |
| 196) Phenyl | 2-Chlor-6-methyl | (4-Methylphenyl)methyl | -CH₂- |
| 197) Phenyl | 2,6-Dichlor | (3-Methylphenyl)methyl | -CH₂- |
| 198) Phenyl | 2-Chlor-6-fluor | (3-Methylphenyl)methyl | -CH₂- |
| 199) Phenyl | 2,6-Difluor | (3-Methylphenyl)methyl | -CH₂- |
| 200) Phenyl | 2-Chlor-5,6-difluor | (3-Methylphenyl)methyl | -CH₂- |
| 201) Phenyl | 2-Chlor-6-trifluormethyl | (3-Methylphenyl)methyl | -CH₂- |
| 202) Phenyl | 2-Fluor-6-trifluormethyl | (3-Methylphenyl)methyl | -CH₂- |
| 203) Phenyl | 2-Brom-6-trifluormethyl | (3-Methylphenyl)methyl | -CH₂- |
| 204) Phenyl | 2-lod-6-trifluormethyl | (3-Methylphenyl)methyl | -CH₂- |
| 205) Phenyl | 2,6-Dibrom | (3-Methylphenyl)methyl | -CH₂- |
| 206) Phenyl | 2-Brom-6-fluor | (3-Methylphenyl)methyl | -CH₂- |
| 207) Phenyl | 2-Brom-6-chlor | (3-Methylphenyl)methyl | -CH₂- |
| 208) Phenyl | 2-Chlor-6-trifluormethoxy | (3-Methylphenyl)methyl | -CH₂- |
| 209) Phenyl | 2-Fluor-6-trifluormethoxy | (3-Methylphenyl)methyl | -CH₂- |
| 210) Phenyl | 2-Chlor-6-difluormethoxy | (3-Methylphenyl)methyl | -CH₂- |
| 211) Phenyl | 2-Difluormethoxy-6-fluor | (3-Methylphenyl)methyl | -CH₂- |
| 212) Phenyl | 2,3-Dichlor-6-difluormethoxy | (3-Methylphenyl)methyl | -CH₂- |
| 213) Phenyl | 2,3-Difluor-6-difluormethoxy | (3-Methylphenyl)methyl | -CH₂- |
| 214) Phenyl | 2,6-Bis(difluormetnoxy) | (3-Methylphenyl)methyl | -CH₂- |
| 215) Phenyl | 2,6-Bis(trfluormethoxy) | (3-Methylphenyl)methyl | -CH₂- |
| 216) Phenyl | 2,6-Bis(trfluormethyl) | (3-Methylphenyl)methyl | -CH₂- |
| 217) Phenyl | 2-Brom | (3-Methylphenyl)methyl | -CH₂- |
| 218) Phenyl | 2-Chlor | (3-Methylphenyl)methyl | -CH₂- |
| 219) Phenyl | 2-Fluor | (3-Methylphenyl)methyl | -CH₂- |
| 220) Phenyl | 3-Brom | (3-Methylphenyl)methyl | -CH₂- |
| 221) Phenyl | 3-Chlor | (3-Methylphenyl)methyl | -CH₂- |
| 222) Phenyl | 3-Fluor | (3-Methylphenyl)methyl | -CH₂- |
| 223) Phenyl | 4-Brom | (3-Methylphenyl)methyl | -CH₂- |
| 224) Phenyl | 4-Chlor | (3-Methylphenyl)methyl | -CH₂- |
| 225) Phenyl | 4-Fluor | (3-Methylphenyl)methyl | -CH₂- |
| 226) Phenyl | 4-Methoxy | (3-Methylphenyl)methyl | -CH₂- |
| 227) Phenyl | 2-Chlor-6-methylthio | (3-Methylphenyl)methyl | -CH₂- |
| 228) Phenyl | 2,3-Difluor-6-methylthio | (3-Methylphenyl)methyl | -CH₂- |
| 229) Phenyl | 2,4-Dichlor | (3-Methylphenyl)methyl | -CH₂- |
| 230) Phenyl | 3,5-Dichlor | (3-Methylphenyl)methyl | -CH₂- |
| 231) Phenyl | 2,3,6-Trichlor | (3-Methylphenyl)methyl | -CH₂- |
| 232) Phenyl | 2,3,6-Trifluor | (3-Methylphenyl)methyl | -CH₂- |
| 233) Phenyl | 2,3,4,5,6-Pentafluor | (3-Methylphenyl)methyl | -CH₂- |
| 234) Phenyl | 2-Fluor-6-methyl | (3-Methylphenyl)methyl | -CH₂- |
| 235) Phenyl | 2-Chlor-6-methyl | (3-Methylphenyl)methyl | -CH₂- |
| 236) Phenyl | 2,6-Dichlor | (4-Trifluormethylphenyl)-methyl | -CH₂- |
| 237) Phenyl | 2-Chlor-6-fluor | (4-Trifluormethylphenyl)-methyl | -CH₂- |
| 238) Phenyl | 2,6-Difluor | (4-Trifluormethylphenyl)-methyl | -CH₂- |
| 239) Phenyl | 2-Chlor-5,6-difluor | (4-Trifluormethylphenyl)-methyl | -CH₂- |
| 240) Phenyl | 2-Chlor-6-trifluormethyl | (4-Trifluormethylphenyl)-methyl | -CH₂- |
| 241) Phenyl | 2-Fluor-6-trifluormethyl | (4-Trifluormethylphenyl)-methyl | -CH₂- |
| 242) Phenyl | 2-Brom-6-trifluormethyl | (4-Trifluormethylphenyl)-methyl | -CH₂- |
| 243) Phenyl | 2-lod-6-trifluormethyl | (4-Trifluormethylphenyl)-methyl | -CH₂- |
| 244) Phenyl | 2,6-Dibrom | (4-Trifluormethylphenyl)-methyl | -CH₂- |
| 245) Phenyl | 2-Brom-6-fluor | (4-Trifluormethylphenyl)-methyl | -CH₂- |
| 246) Phenyl | 2-Brom-6-chlor | (4-Trifluormethylphenyl)-methyl | -CH₂- |
| 247) Phenyl | 2-Chlor-6-trifluormethoxy | (4-Trifluormethylphenyl)-methyl | -CH₂- |
| 248) Phenyl | 2-Fiuor-6-trifluormethoxy | (4-Trifluormethylphenyl)-methyl | -CH₂- |
| 249) Phenyl | 2-Chlor-6-difluormethoxy | (4-Trifluormethylphenyl)-methyl | -CH₂- |
| 250) Phenyl | 2-Difluormethoxy-6-fluor | (4-Trifluormethylphenyl)-methyl | -CH₂- |
| 251) Phenyl | 2,3-Dichlor-6-difluormethoxy | (4-Trifluormethylphenyl)-methyl | -CH₂- |
| 252) Phenyl | 2,3-Difluor-6-difluormethoxy | (4-Trifluormethylphenyl)-methyl | -CH₂- |
| 253) Phenyl | 2,6-Bis(difluormethoxy) | (4-Trifluormethylphenyl)-methyl | -CH₂- |
| 254) Phenyl | 2,6-Bis(trfluormethoxy) | (4-Trifluormethylphenyl)-methyl | -CH₂- |
| 255) Phenyl | 2,6-Bis(trfluormethyl) | (4-Trifluormethylphenyl)-methyl | -CH₂- |
| 256) Phenyl | 2-Brom | (4-Trifluormethylphenyl)-methyl | -CH₂- |
| 257) Phenyl | 2-Chlor | (4-Trifluormethylphenyl)-methyl | -CH₂- |
| 258) Phenyl | 2-Fluor | (4-Trifluormethylphenyl)-methyl | -CH₂- |
| 259) Phenyl | 3-Brom | (4-Trifluormethylphenyl)-methyl | -CH₂- |
| 260) Phenyl | 3-Chlor | (4-Trifluormethylphenyl)-methyl | -CH₂- |
| 261) Phenyl | 3-Fluor | (4-Trifluormethylphenyl)-methyl | -CH₂- |
| 262) Phenyl | 4-Brom | (4-Trifluormethylphenyl)-methyl | -CH₂- |
| 263) Phenyl | 4-Chlor | (4-Trifluormethylphenyl)-methyl | -CH₂- |
| 264) Phenyl | 4-Fluor | (4-Trifluormethylphenyl)-methyl | -CH₂- |
| 265) Phenyl | 4-Methoxy | (4-Trifluormethylphenyl)-methyl | -CH₂- |
| 266) Phenyl | 2-Chlor-6-methylthio | (4-Trifluormethylphenyl)-methyl | -CH₂- |
| 267) Phenyl | 2,3-Difluor-6-methylthio | (4-Trifluormethylphenyl)-methyl | -CH₂- |
| 268) Phenyl | 2,4-Dichlor | (4-Trifluormethylphenyl)-methyl | -CH₂- |
| 269) Phenyl | 3,5-Dichlor | (4-Trifluormethylphenyl)-methyl | -CH₂- |
| 270) Phenyl | 2,3,6-Trichlor | (4-Trifluormethylphenyl)-methyl | -CH₂- |
| 271) Phenyl | 2,3,6-Trifluor | (4-Trifluormethylphenyl)-methyl | -CH₂- |
| 272) Phenyl | 2,3,4,5,6-Pentafluor | (4-Trifluormethylphenyl)-methyl | -CH₂- |
| 273) Phenyl | 2-Fluor-6-methyl | (4-Trifluormethylphenyl)-methyl | -CH₂- |
| 274) Phenyl | 2-Chlor-6-methyl | (4-Trifluormethylphenyl)-methyl | -CH₂- |
| 275) Phenyl | 2,6-Dichlor | (4-Methoxyphenyl)methyl | -CH₂- |
| 276) Phenyl | 2-Chlor-6-fluor | (4-Methoxyphenyl)methyl | -CH₂- |
| 277) Phenyl | 2,6-Difluor | (4-Methoxyphenyl)methyl | -CH₂- |
| 278) Phenyl | 2-Chlor-5,6-difluor | (4-Methoxyphenyl)methyl | -CH₂- |
| 279) Phenyl | 2-Chlor-6-trifluormethyl | (4-Methoxyphenyl)methyl | -CH₂- |
| 280) Phenyl | 2-Fluor-6-trifluormethyl | (4-Methoxyphenyl)methyl | -CH₂- |
| 281) Phenyl | 2-Brom-6-trifluormethyl | (4-Methoxyphenyl)methyl | -CH₂- |
| 282) Phenyl | 2-lod-6-trifluormethyl | (4-Methoxyphenyl)methyl | -CH₂- |
| 283) Phenyl | 2,6-Dibrom | (4-Methoxyphenyl)methyl | -CH₂- |
| 284) Phenyl | 2-Brom-6-fluor | (4-Methoxyphenyl)methyl | -CH₂- |
| 285) Phenyl | 2-Brom-6-chlor | (4-Methoxyphenyl)methyl | -CH₂- |
| 286) Phenyl | 2-Chlor-6-trifluormethoxy | (4-Methoxyphenyl)methyl | -CH₂- |
| 287) Phenyl | 2-Fluor-6-trifluormethoxy | (4-Methoxyphenyl)methyl | -CH₂- |
| 288) Phenyl | 2-Chlor-6-difluormethoxy | (4-Methoxyphenyl)methyl | -CH₂- |
| 289) Phenyl | 2-Difluormethoxy-6-fluor | (4-Methoxyphenyl)methyl | -CH₂- |
| 290) Phenyl | 2,3-Dichlor-6-difluormethoxy | (4-Methoxyphenyl)methyl | -CH₂- |
| 291) Phenyl | 2,3-Difluor-6-difluormethoxy | (4-Methoxyphenyl)methyl | -CH₂- |
| 292) Phenyl | 2,6-Bis(difluormethoxy) | (4-Methoxyphenyl)methyl | -CH₂- |
| 293) Phenyl | 2,6-Bis(trfluormethoxy) | (4-Methoxyphenyl)methyl | -CH₂- |
| 294) Phenyl | 2,6-Bis(trfluormethyl) | (4-Methoxyphenyl)methyl | -CH₂- |
| 295) Phenyl | 2-Brom | (4-Methoxyphenyl)methyl | -CH₂- |
| 296) Phenyl | 2-Chlor | (4-Methoxyphenyl)methyl | -CH₂- |
| 297) Phenyl | 2-Fluor | (4-Methoxyphenyl)methyl | -CH₂- |
| 298) Phenyl | 3-Brom | (4-Methoxyphenyl)methyl | -CH₂- |
| 299) Phenyl | 3-Chlor | (4-Methoxyphenyl)methyl | -CH₂- |
| 300) Phenyl | 3-Fluor | (4-Methoxyphenyl)methyl | -CH₂- |
| 301) Phenyl | 4-Brom | (4-Methoxyphenyl)methyl | -CH₂- |
| 302) Phenyl | 4-Chlor | (4-Methoxyphenyl)methyl | -CH₂- |
| 303) Phenyl | 4-Fluor | (4-Methoxyphenyl)methyl | -CH₂- |
| 304) Phenyl | 4-Methoxy | (4-Methoxyphenyl)methyl | -CH₂- |
| 305) Phenyl | 2-Chlor-6-methylthio | (4-Methoxyphenyl)methyl | -CH₂- |
| 306) Phenyl | 2,3-Difluor-6-methylthio | (4-Methoxyphenyl)methyl | -CH₂- |
| 307) Phenyl | 2,4-Dichlor | (4-Methoxyphenyl)methyl | -CH₂- |
| 308) Phenyl | 3,5-Dichlor | (4-Methoxyphenyl)methyl | -CH₂- |
| 309) Phenyl | 2,3,6-Trichlor | (4-Methoxyphenyl)methyl | -CH₂- |
| 310) Phenyl | 2,3,6-Trifluor | (4-Methoxyphenyl)methyl | -CH₂- |
| 311) Phenyl | 2,3,4,5,6-Pentafluor | (4-Methoxyphenyl)methyl | -CH₂- |
| 312) Phenyl | 2-Fluor-6-methyl | (4-Methoxyphenyl)methyl | -CH₂- |
| 313) Phenyl | 2-Chlor-6-methyl | (4-Methoxyphenyl)methyl | -CH₂- |
| 314) Phenyl | 2,6-Dichlor | (2-Thienyl)methyl | -CH₂- |
| 315) Phenyl | 2-Chlor-6-fluor | (2-Thienyl)methyl | -CH₂- |
| 316) Phenyl | 2,6-Difluor | (2-Thienyl)methyl | -CH₂- |
| 317) Phenyl | 2-Chlor-5,6-difluor | (2-Thienyl)methyl | -CH₂- |
| 318) Phenyl | 2-Chlor-6-trifluormethyl | (2-Thienyl)methyl | -CH₂- |
| 319) Phenyl | 2-Fluor-6-trifluormethyl | (2-Thienyl)methyl | -CH₂- |
| 320) Phenyl | 2-Brom-6-trifluormethyl | (2-Thienyl)methyl | -CH₂- |
| 321) Phenyl | 2-lod-6-trifluormethyl | (2-Thienyl)methyl | -CH₂- |
| 322) Phenyl | 2,6-Dibrom | (2-Thienyl)methyl | -CH₂- |
| 323) Phenyl | 2-Brom-6-fluor | (2-Thienyl)methyl | -CH₂- |
| 324) Phenyl | 2-Brom-6-chlor | (2-Thienyl)methyl | -CH₂- |
| 325) Phenyl | 2-Chlor-6-trifluormethoxy | (2-Thienyl)methyl | -CH₂- |
| 326) Phenyl | 2-Fluor-6-trifluormethoxy | (2-Thienyl)methyl | -CH₂- |
| 327) Phenyl | 2-Chlor-6-difluormethoxy | (2-Thienyl)methyl | -CH₂- |
| 328) Phenyl | 2-Difluormethoxy-6-fluor | (2-Thienyl)methyl | -CH₂- |
| 329) Phenyl | 2,3-Dichlor-6-difluormethoxy | (2-Thienyl)methyl | -CH₂- |
| 330) Phenyl | 2,3-Difluor-6-difluormethoxy | (2-Thienyl)methyl | -CH₂- |
| 331) Phenyl | 2,6-Bis(difluormethoxy) | (2-Thienyl)methyl | -CH₂- |
| 332) Phenyl | 2,6-Bis(trfluormethoxy) | (2-Thienyl)methyl | -CH₂- |
| 333) Phenyl | 2,6-Bis(trfluormethyl) | (2-Thienyl)methyl | -CH₂- |
| 334) Phenyl | 2-Brom | (2-Thienyl)methyl | -CH₂- |
| 335) Phenyl | 2-Chlor | (2-Thienyl)methyl | -CH₂- |
| 336) Phenyt | 2-Fluor | (2-Thienyl)methyl | -CH₂- |
| 337) Phenyl | 3-Brom | (2-Thienyl)methyl | -CH₂- |
| 338) Phenyl | 3-Chlor | (2-Thienyl)methyl | -CH₂- |
| 339) Phenyl | 3-Fluor | (2-Thlenyl)methyl | -CH₂- |
| 340) Phenyl | 4-Brom | (2-Thienyl)methyl | -CH₂- |
| 341) Phenyl | 4-Chlor | (2-Thienyl)methyl | -CH₂- |
| 342) Phenyl | 4-Fluor | (2-Thienyl)methyl | -CH₂- |
| 343) Phenyl | 4-Methoxy | (2-Thienyl)methyl | -CH₂- |
| 344) Phenyl | 2-Chlor-6-methylthio | (2-Thienyl)methyl | -CH₂- |
| 345) Phenyl | 2,3-Difluor-6-methylthio | (2-Thienyl)methyl | -CH₂- |
| 346) Phenyl | 2,4-Dichlor | (2-Thienyl)methyl | -CH₂- |
| 347) Phenyl | 3,5-Dichlor | (2-Thienyl)methyl | -CH₂- |
| 348) Phenyl | 2,3,6-Trichlor | (2-Thienyl)methyl | -CH₂- |
| 349) Phenyl | 2,3,6-Trifluor | (2-Thienyl)metnyt | -CH₂- |
| 350) Phenyl | 2,3,4,5,6-Pentafluor | (2-Thienyl)methyl | -CH₂- |
| 351) Phenyl | 2-Fluor-6-methyl | (2-Thienyl)methyl | -CH₂- |
| 352) Phenyl | 2-Chlor-6-methyl | (2-Thlenyl)methyl | -CH₂- |
| 353) Phenyl | 2,6-Dichlor | Phenylmethyl | -CH(CH₃)- |
| 354) Phenyl | 2-Chlor-6-fluor | Phenylmethyl | -CH(CH₃)- |
| 355) Phenyl | 2,6-Difluor | Phenylmethyl | -CH(CH₃)- |
| 356) Phenyl | 2-Chlor-5,6-difluor | Phenylmethyl | -CH(CH₃)- |
| 357) Phenyl | 2-Chlor-6-trifluormethyl | Phenylmethyl | -CH(CH₃)- |
| 358) Phenyl | 2-Fluor-6-trifluormethyl | Phenylmethyl | -CH(CH₃)- |
| 359) Phenyl | | Phenylmethyl | -CH(CH₃)- |
| 360) Phenyl | 2,6-Dichlor | Phenylmethyl | -O-CH₂- |
| 361) Phenyl | 2-Chlor-6-fluor | Phenylmethyl | -O-CH₂- |
| 362) Phenyl | 2,6-Difluor | Phenylmethyl | -O-CH₂- |
| 363) Phenyl | 2-Chlor-5,6-difluor | Phenylmethyl | -O-CH₂- |
| 364) Phenyl | 2-Chlor-6-trifluormethyl | Phenylmethyl | -O-CH₂- |
| 365) Phenyl | 2-Fluor-6-trifluormethyl | Phenylmethyl | -O-CH₂- |
| 366) Phenyl | | Phenylmethyl | -O-CH₂- |
| 367) 2-Pyridyl | | Phenylmethyl | -CH₂- |
| 368) 3-Pyridyl | | Phenylmethyl | -CH₂- |
| 369) 2-Pyridyl | 3-Chlor | Phenylmethyl | -CH₂- |
| 370) 2-Thienyl | 3-Chlor | Phenylmethyl | -CH₂- |
| 371) 3-Pyridyl | 4-Chlor | Phenylmethyl | -CH₂- |
| 372) 3-Pyridyl | 4-Trifluormethyl | Phenylmethyl | -CH₂- |
| 373) 3-Pyridyl | 2-Methyl-4-trifluormethyl | Phenylmethyl | -CH₂- |
| 374) Phenyl | 2,3-Dichlor | Phenylmethyl | -CH₂- |

Die Amidoxime der Formel III werden durch Umsetzung von Nitrilen der Formel II mit Hydroxylamin oder dessen Salzen in wäßriger Lösung, vorzugsweise in Wasser oder Wasser/Alkanol-Gemischen, gegebenenfalls in Anwesenheit einer Base gewonnen. Diese können dann in an sich bekannter Weise zu den Vorprodukten IV alkyliert werden, wobei man als Alkylierungsmittel vorzugsweise Cyclopropylmethylbromid oder Cyclopropylmethylchlorid verwendet. Auch das Iodid oder organische Sulfonsäurereste kommen zur Aktivierung des Cyclopropylmethylrestes infrage.

Die Verbindungen der Formel 1 lassen sich bevorzugt nach dem folgenden Schema darstellen:

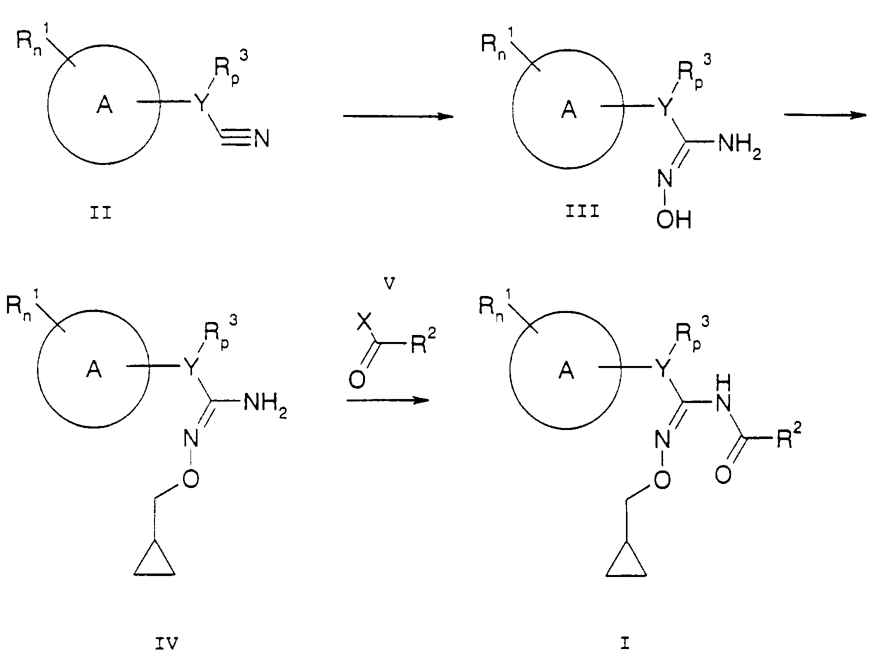

Die Amidoxime IV können dann in an sich bekannter Weise mit den entsprechenden Säurederivaten V, vorzugsweise den entsprechenden Säurechloriden oder Säureanhydriden, durch Erwärmen in inerten Lösungsmitteln (vorzugsweise auf Temperaturen im Bereich von 20 bis 100 °C) acyliert werden. Als inerte Lösungsmittel eignen sich insbesondere Kohlenwasserstoffe oder Ether, besonders bevorzugt aromatische Kohlenwasserstoffe wie Toluol oder Xylol, um nur zwei Beispiele zu nennen.

Die in vorstehendem Reaktionsschema aufgeführten Zwischenprodukte der Formel III und die Zwischenprodukte der Formel IV sind neu und ebenfalls Gegenstand der vorliegenden Erfindung.
Bevorzugte Amidoxime der Formel III sind die in Tabelle 2 genannten Verbindungen:

**Tabelle 2:**

| A | R¹ₙ | Y-R³ₚ | Physikal. Daten |
|---|---|---|---|
| | | | |
| Phenyl | 2,6-Dichlor | -CH₂- | Schmp. 172-173°C |
| Phenyl | 2-Chlor-6-fluor | -CH₂- | Schmp. 138-141°C |
| Phenyl | 2,3,6-Trifluor | -CH₂- | Schmp. 151-153°C |
| Phenyl | | -CH₂- | Schmp. 39-42°C |
| Phenyl | | -CH(CH₃)- | Schmp. 85-88°C |
| Phenyl | 2,6-Difluor | -CH₂- | Schmp. 124-126°C |
| Phenyl | 3, 5-Dichlor | -CH₂- | Schmp. 103-107°C |
| Phenyl | 2, 3-Dichlor | -CH₂- | Schmp. 162-163°C |
| Phenyl | 2, 3, 6-Trichlor | -CH₂- | ¹H-NMR (CDCl₃) δ = 3,90 (s); 4,63 (s); 7,25-7,40 (m);7,43 (verbr.). |
| Phenyl | 2-Fluor-6-trifluormethyl | -CH₂- | ¹H-NMR (CDCl₃) δ = 3,72 (s); 4,58 (s); 7,20-7,50 (m). |
| Phenyl | 2-Chlor | -CH₂- | ¹H-NMR (CDCl₃) δ = 3,63 (s); 4,63 (s); 7,22 (m); 7,35 (m); 8,67 (verbr.). |
| Phenyl | 2,4-Dichlor | -CH₂- | Schmp. 155-157°C |

Bevorzugte Amidoxim-Derivate der Formel I sind die in Tabelle 3 genannten Verbindungen, wobei R² für Benzyl steht:

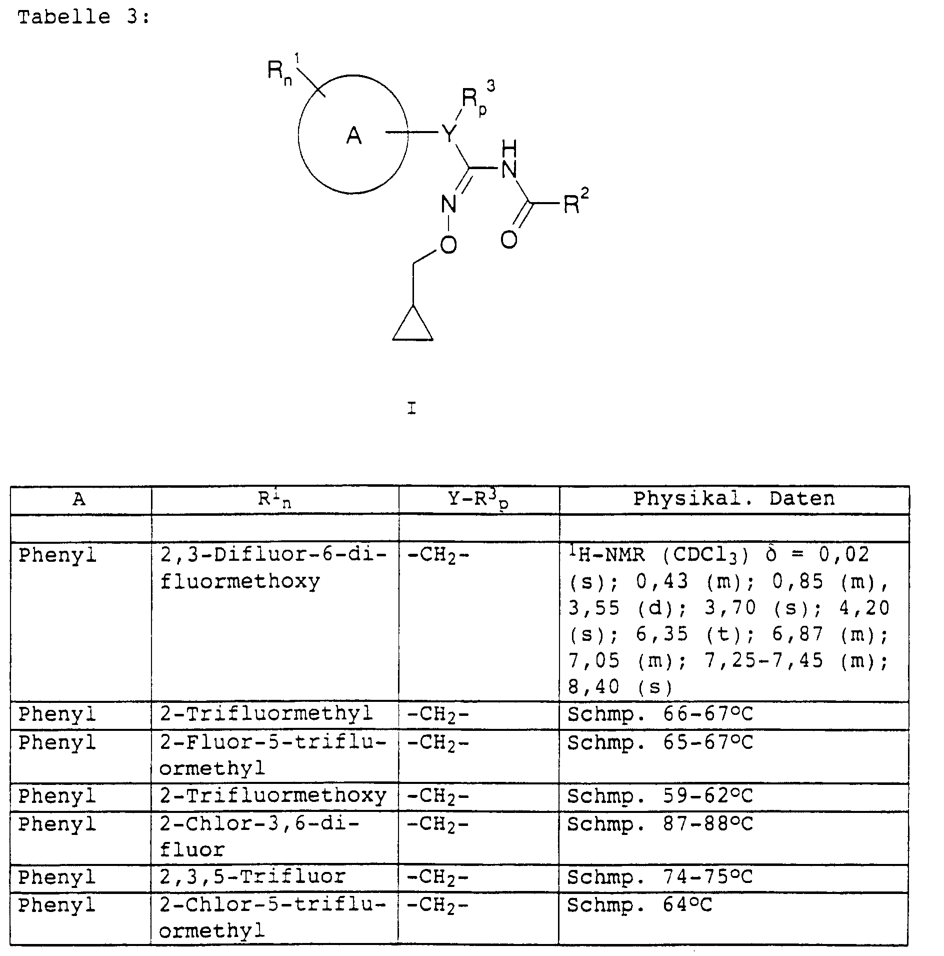

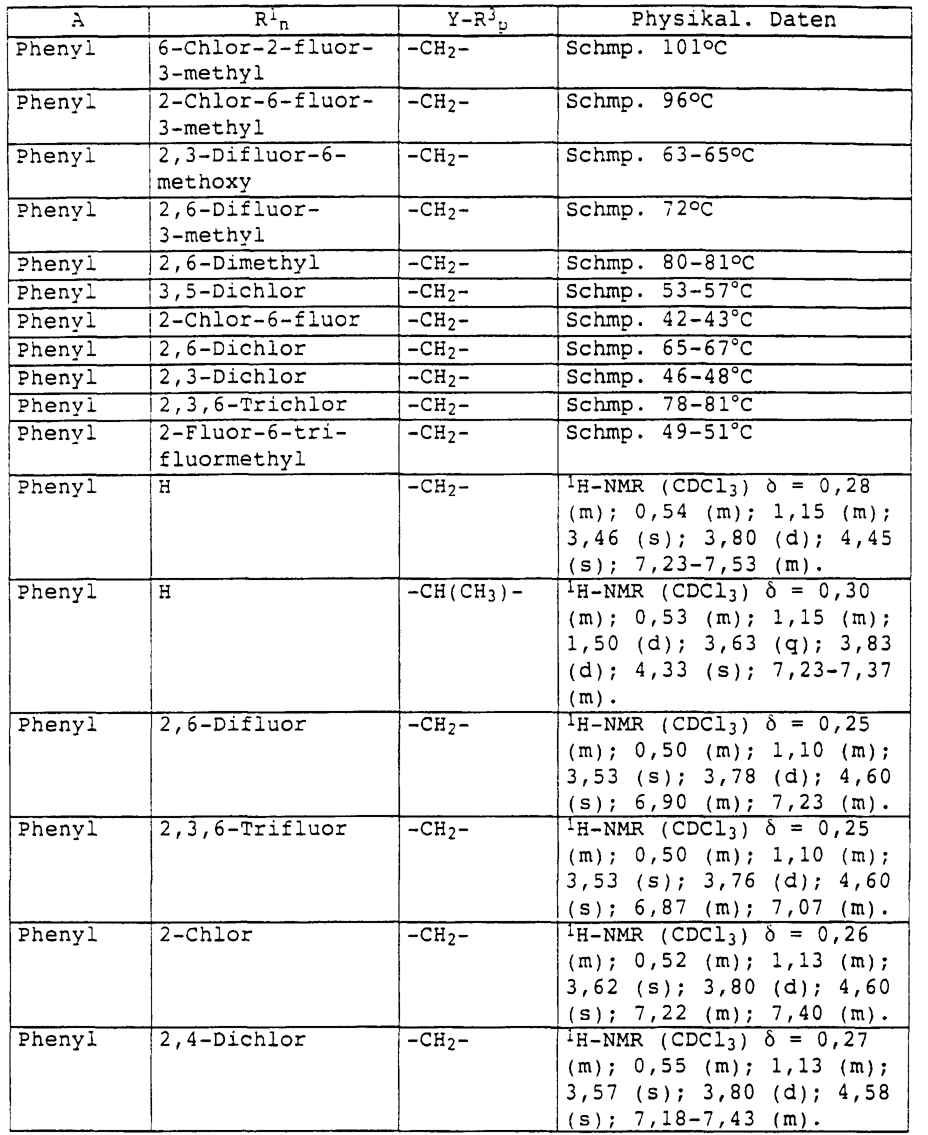

Die Verbindungen I zeichnen sich durch eine hervorragende Wirkung gegen ein breites Spektrum von pflanzenpathogenen Pilzen, insbesondere aus der Klasse der Ascomyceten, Deuteromyceten, Phycomyceten und Basidiomyceten, aus. Sie sind z.T. systemisch wirksam und können daher auch als Blatt- und Bodenfungizide eingesetzt werden.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calciumund Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 10 Gew.-Teilen einer erfindungsgemäßen Verbindung I, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III.eine wäßrige Dispersion aus 10 Gew.-Teilen einer erfindungsgemäßen Verbindung I, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen einer erfindungsgemäßen Verbindung I, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen, vorzugsweise einer festen erfindungsgemäßen Verbindung I, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII.eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Caiciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfid, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(ptert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
   verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,

Strobilurine wie Methyl-E-methoximino-[a-(o-tolyloxy)-o-tolyl]acetat, Methyl-E-2-{2-[6-(2-cyanophenoxy)pyridimin-4-yloxy]phenyl}-3-methoxyacrylat, Methyl-E-methoximino-[a-(2,5-dimethylphenoxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)-pyrrol-3-carbonitril.

Zimtsäureamide wie 3-(4-chlorphenyl)-3-(3,4-dimethoxyphenyl)acrylsäuremorpholid.

### Beispiel 1

### O-Cyclopropylmethyl-N-phenylacetyl-(2,6-dichlorphenyl)-acetamidoxim (Verbindung Nr. 1 aus Tabelle 1)

### a) (2,6-Dichlorphenyl)acetamidoxim

Zu 15,0 g (81 mmol) (2,6-Dichlorphenyl)acetonitril in 60 ml Ethanol wurden 10,3 g (148 mmol) Hydroxylaminhydrochlorid und anschließend 11,1 g (105 mmol) Natriumcarbonat gelöst in 40 ml Wasser gegeben. Diese Mischung wurde 4 h unter Rückfluß gekocht, in wässrigen Natriumdihydrogenphosphat-Puffer (pH 7-8) gegeben und mit Methylenchlorid extrahiert. Der dabei aus gefallene weiße Feststoff (14,0 g) wurde abfiltriert und im Vakuum getrocknet. Weiteres Produkt (3,1 g) wurde nach dem Entfernen des Lösungsmittels im Vakuum aus dem Extrakt gewonnen. Insgesamt betrug die Ausbeute 17,1 g mit dem Schmp. 172-173°C.

### b) O-Cyclopropylmethyl-(2,6-dichlorphenyl)acetamidoxim

Zu 10,0 g (46 mmol) (2,6-Dichlorphenyl)acetamidoxim in 40 ml Dimethylformamid wurden 6,5 g (48 mmol) Cyclopropylmethylbromid gegeben. Die Mischung wurde auf -20°C gekühlt und tropfenweise mit 5,4 g (48 mmol) Kalium-tert.-butylat in 20 ml Dimethylformamid versetzt. Die Mischung wurde 1 h bei -20°C und über Nacht bei Raumtemperatur gerührt, auf wässrigen Natriumdihydrogenphosphat-Puffer gegossen (pH 6) und 5 mal mit Diethylether extrahiert. Die vereinigten Extrakte wurden zweimal mit Wasser und einmal mit gesättigter Kochsalz-Lösung gewaschen, über Natriumsulfat getrocknet und im Vakuum eingeengt. Ausbeute 12,3 g gelbes Öl, das ohne weitere Reinigung weiter umgesetzt wurde.

### c) O-Cyclopropylmethyl-N-phenylacetyl-(2,6-dichlorphenyl)-acetamidoxim

5,0 g (18 mmol) O-Cyclopropylmethyl-(2,6-dichlorphenyl)acetamidoxim in 40 ml Toluol wurden auf 85°C erwärmt und mit 3,9 g (25 mmol) Phenylacetylchlorid versetzt. Die Mischung wurde 5 h auf 100°C erwärmt, abgekühlt, auf wässrige Natriumhydrogencarbonat-Lösung gegeben (pH 7) und mit dreimal Toluol extrahiert. Die vereinigten Extrakte wurden mit Wasser gewaschen, über Natriumsulfat getrocknet und im Vakuum eingeengt. Das Rohprodukt (5,6 g) wurde durch Chromatographie an Kieselgel mit Cyclohexan/Essigsäureethylester gereinigt. Schmp. 134-135°C.

### Beispiel 2

### O-Cyclopropylmethyl-N-phenylacetyl-(2-chlor-6-fluorphenyl)acetamidoxim (Verbindung Nr. 2 aus Tabelle 1)

### a) (2-Chlor-6-fluorphenyl)acetamidoxim

Zu 10,0 g (59 mmol) (2-Chlor-6-fluorphenyl)acetonitril) in 50 ml Ethanol wurden 7,0 g (101 mmol) Hydroxylaminhydrochlorid und anschließend 7,5 g (71 mmol) Natriumcarbonat gelöst in 30 ml Wasser gegeben. Diese Mischung wurde 4 h unter Rückfluß gekocht, in wässrigen Natriumdihydrogenphosphat-Puffer (pH 7-8) gegeben, mit Methylenchlorid extrahiert und über Natriumsulfat getrocknet. Nach dem Entfernen des Lösungsmittels im Vakuum wurden aus dem Extrakt 4,9 g Produkt gewonnen. Weitere 3,7 g fielen aus der wässrigen Phase aus. Ausbeute insgesamt: 8,6 g, die direkt weiter umgesetzt wurden.

### b) 0-Cyclopropylmethyl-(2-chlor-6-fluorphenyl)acetamidoxim

Zu 4,0 g (20 mmol) (2-Chlor-6-fluorphenyl)acetamidoxim in 30 ml Dimethylformamid wurden 2,8 g (21 mmol) Cyclopropylmethylbromid gegeben. Die Mischung wurde auf -20°C gekühlt und tropfenweise mit 2,4 g (21 mmol) Kalium-tert.-butylat in 20 ml Dimethylformamid versetzt. Die Mischung wurde 1 h bei -20°C und über Nacht bei Raumtemperatur gerührt, auf wässrigen Natriumdihydrogenphosphat-Puffer gegossen (pH 6) und 5 mal mit Diethylether extrahiert. Die vereinigten Extrakte wurden zweimal mit Wasser und einmal mit gesättigter Kochsalz-Lösung gewaschen, über Natriumsulfat getrocknet und im Vakuum eingeengt. Ausbeute 4,8 g gelbes Öl, das ohne weitere Reinigung weiter umgesetzt wurde.

### c) O-Cyclopropylmethyl-N-phenylacetyl-(2-chlor-6-fluorphenyl)-acetamidoxim

3,0 g (12 mmol) O-Cyclopropylmethyl-(2-chlor-6-fluorphenyl)-acetamidoxim in 30 ml Toluol wurden auf 85°C erwärmt und mit 2,5 g (16 mmol) Phenylacetylchlorid versetzt. Die Mischung wurde 5 h auf 100°C erwärmt, abgekühlt, auf wässrige Natriumhydrogen-carbonat-Lösung gegeben (pH 7) und mit dreimal Toluol extrahiert. Die vereinigten Extrakte wurden mit Wasser gewaschen, über Natriumsulfat getrocknet und im Vakuum eingeengt. Das Rohprodukt (3,8 g) wurde durch Chromatographie an Kieselgel mit Cyclohexan/Essigsäureethylester gereinigt. Ausbeute 1,5 g vom Schmp. 109-110°C.

### Beispiel 3

Analog zu den in den Beispielen 1 und 2 beschriebenen Methoden wurden die folgenden Verbindungen hergestellt:

| Verbindung aus Tabelle 1 | Physikalische Daten |
|---|---|
| | |
| Nr. 3 | Schmp. 75-78°C |
| Nr. 5 | ¹H-NMR (CDCl₃) δ = 0,17 (m); 0, 48 (m); 0,97 (m); 3,56 (s); 3,75 (d); 4,03 (s); 7,10-7,25 (m); 8,23 (s). |
| Nr. 7 | ¹H-NMR (CDCl₃) δ = -0,05 (m); 0,35 (m); 0,79 (m); 3,50 (d); 3,73 (s); 4,32 (s); 7,10-7,45 (m); 8,43 (s). |
| Nr. 23 | Schmp. 69-72°C |
| Nr. 34 | Schmp. 94-96°C |
| Nr. 35 | Schmp. 76-80°C |
| Nr. 36 | Schmp. 95-98°C |
| Nr. 37 | Schmp. 58-61°C |
| Nr. 359 | ¹H-NMR (CDCl₃) δ = 0,20 (m); 0, 48 (m); 1,00 (m); 1,38 (d); 3,50 (m); 3,78 (d); 4,87 (q); 7,05-7,35 (m); 8,19 (s). |
| Nr. 374 | Schmp. 63-65°C |

### Beispiel 4

### Wirksamkeit gegen Weizenmehltau

Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (*Erysiphe graminis* forma specialis *tritici*) bestäubt. Die Versuchspflanzen wurden anschließend im Gewächshaus bei Temperaturen zwischen 20 und 24° C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

| Wirkstoff Nr. aus Tabelle 1 | % - Befall der Blätter nach Applikation von 16 ppm-haltiger wäßriger Wirkstoffaufbereitung |
|---|---|
| Nr. 1 | 3 |
| Nr. 2 | 3 |
| Unbehandelt | 95 |

Die mit den Wirkstoffen Nr. 1 und 2 der Tabelle 1 behandelten Pflanzen zeigten einen Befall von nur 3 %, während die unbehandelten Pflanzen zu 95 % befallen waren.

### Beispiel 5

### Protektive Wirksamkeit gegen Gurkenmehltau

Blätter von in Töpfen gewachsenen Gurkenkeimlingen der Sorte "Chinesische Schlange" wurden im Zweiblattstadium mit wäßriger Wirkstoffaufbereitung, die mit einer Stammlösung aus 10 % Wirkstoff, 63 % Cyclohexanon und 27 % Emulgiermittel angesetzt wurde, bis zur Tropfnässe besprüht. 20 Stunden nach dem Antrocknen des Spritzbelages wurden die Pflanzen mit einer wäßrigen Sporensuspension des Gurkenmehltaus (*Sphaerotheca fuliginea*) inokuliert. Anschließend wurden die Pflanzen im Gewächshaus bei Temperaturen zwischen 20 und 24° C und 60 bis 80 % relativer Luftfeuchtigkeit für 20 Tage kultiviert. Dann wurde das Ausmaß der Mehltauentwicklung visuell in %-Befall der gesamten Blattfläche ermittelt.

| Wirkstoff Nr. aus Tabelle 1 | % - Befall der Blätter nach Applikation von 63 ppm-haltiger wäßriger Wirkstoffaufbereitung |
|---|---|
| Wirkstoff Nr. 1 | 10 |
| Wirkstoff Nr. 2 | 10 |
| Unbehandelt | 90 |

## Patentansprüche

1. Benzylamidoxime-Derivate der Formel I 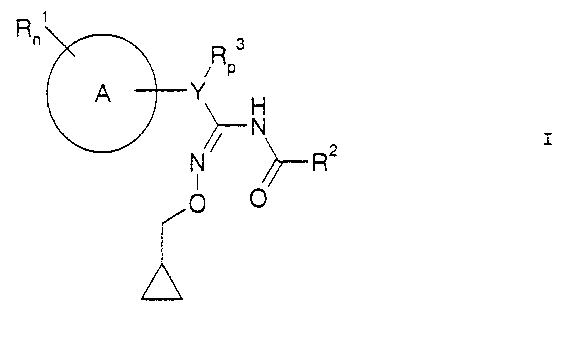 wobei die Reste folgende Bedeutung haben:
A ein Aryl oder Hetarylrest aus der Gruppe Phenyl, Pyridyl oder Thienyl;
Y eine geradkettige oder verzweigte C₁-C₄-Alkylengruppe, wobei ein Kohlenstoffatom durch ein Sauerstoff-, Stickstoff- oder Schwefelatom oder durch eine Cyclopropylgruppe ersetzt sein kann;
Rₙ¹ ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
R² Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazolyl-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann,
Rₚ³ ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy, C₁-C₆-Alkylcarbonyl;
n 0-5;
p je nach Anzahl der freien Valenzen 0-4.

2. Benzylamidoxime der Formel I nach Anspruch 1, wobei A Phenyl bedeutet.

3. Benzylamidoxime der Formel I nach Anspruch 1, wobei A Pyridyl bedeutet.

4. Benzylamidoxime der Formel I nach Anspruch 1 oder 2, wobei Y ein Kohlenstoffatom darstellt.

5. Benzylamidoxime der Formel I nach einem der Ansprüche 1 - 3, wobei Rₙ¹ ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy bedeutet.

6. Benzylamidoxime der Formel I nach einem der Ansprüche 1 - 4, wobei
R² Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazolyl-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann,
bedeutet.

7. Benzylamidoxime der Formel I nach einem der Ansprüche 1 - 5, wobei Rₚ³ ein bis zwei gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy bedeutet.

8. Benzylamidoxime der Formel I nach Anspruch 7, wobei Rₚ³ Wasserstoff oder C₁-C₄-Alkyl bedeutet.

9. Benzylamidoxime der Formel I nach Anspruch 1, wobei die Reste folgende Bedeutung haben:
A ein Aryl oder Hetarylrest aus der Gruppe Phenyl, Pyridyl oder Thienyl;
Y ein Kohlenstoffatom;
Rₙ¹ ein bis fünf gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
R² Phenyl-C₁-C₆-Alkyl, welches am Phenylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Thienyl-C₁-C₄-Alkyl, welches am Thienylring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann, oder
Pyrazolyl-C₁-C₄-Alkyl, welches am Pyrazolring einen oder mehrere Substituenten ausgewählt aus der Gruppe bestehend aus Halogen, C₁-C₄-Alkyl, C₁-C₄-Halogenalkyl, C₁-C₄-Alkoxy oder C₁-C₄-Halogenalkoxy tragen kann,
Rₚ³ ein bis zwei gleiche oder verschiedene Reste aus der Gruppe: Wasserstoff, Halogen, C₁-C₆-Alkyl, C₁-C₆-Alkoxy, C₁-C₄-Halogenalkyl, C₁-C₄-Halogenalkoxy, C₁-C₄-Alkylthio, C₁-C₄-Alkoxyalkoxy;
n 0-5;
p 0-2.

10. Verwendung von Amidoximen der Formel III 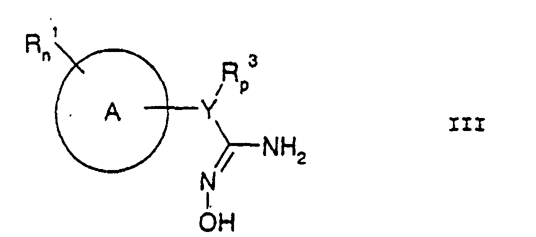 wobei Rₙ¹ und Rₚ³ die in Anspruch 1 angesehenen Bedeutungen haben, zur Herstellung von Amidoxim-Derivaten der Formel I.

11. Amidoxim-Derivate der Formel IV 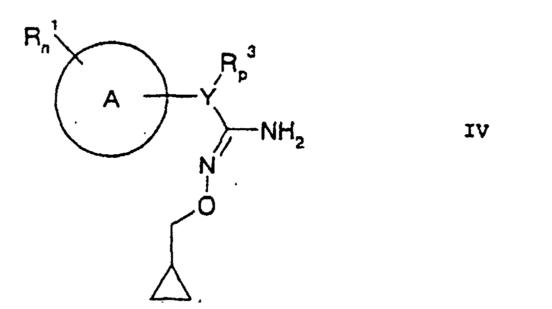 wobei Rₙ¹, Rₚ³, A und Y die in Anspruch 1 angegebenen Bedeutungen haben.

12. Verwendung von Verbindungen der Formel IV gemäß Anspruch 11 zur Herstellung von Benzamidoxim-Derivate der Formel I.

13. Verwendung der Benzamidoxim-Derivate der Formel I gemäß den Ansprüchen 1-9 zur Bekämpfung von Schadpilzen.

14. Verfahren zur Herstellung der Benzamidoxim-Derivate der Formel I gemäß einem der Ansprüche 1-9, **dadurch gekennzeichnet, daß** man Benzonitrile der Formel II 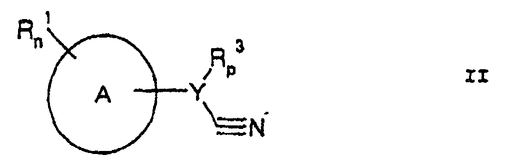 mit Hydroxylamin oder dessen Salzen in wäßriger Lösung, vorzugsweise bei einem pH-Wert von größer 8 zu Benzamidoximen der Formel III 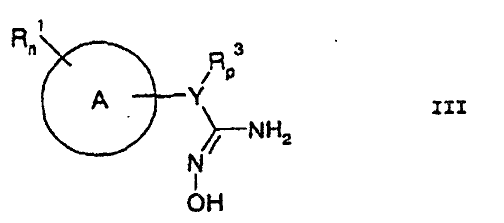 umsetzt, diese anschließend mit einem cyclopropylmethylhalogenid zu Benzamidoximen der Formel IV 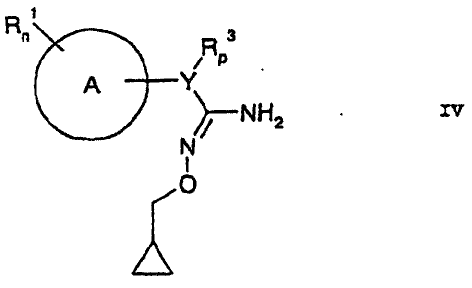 alkyliert und anschließend mit einem entsprechenden säurehalogenid in Benzamidoxim-Derivate der formel I Überführt.

15. Agrochemische Zusammensetzung enthaltend eine fungizid wirksame Menge mindestens eines Benzamidoxim-Derivats der Formel I nach den Ansprüchen 1-9, sowie gegebenenfalls landwirtschaftlich einsetzbare Hilfs- oder Zusatzstoffe.

16. Verfahren zur Bekämpfung von Schadpilzen, **dadurch gekennzeichnet, daß** man die Schadpilze, deren Lebensraum oder die von ihnen freizuhaltenden Pflanzen, Flächen, Materialien oder Räume mit einer fungizid wirksamen Menge einer Verbindung der allgemeinen formel I oder einem ein Benzamidoxim-Derivat der Formel I enthaltenden fungiziden Mittel gemäß Anspruch 15 behandelt.

## Claims

1. A benzamidoxime derivative of the formula I 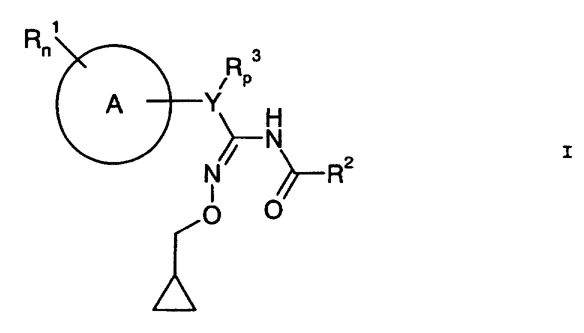 where:
A is an aryl or hetaryl radical from the group consisting of phenyl, pyridyl and thienyl;
Y is a straight-chain or branched C₁-C₄-alkylene group, where one carbon can be replaced by oxygen, nitrogen or sulfur or by a cyclopropyl group;
Rₙ¹ are one to five identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy;
R² is phenyl-C₁-C₆-alkyl, which may carry one or more substituents selected from the group consisting of halogen, C₁-C₄-alkyl, C₁-C₄-haloalkyl, C₁-C₄-alkoxy and C₁-C₄-haloalkoxy on the phenyl ring, or
is thienyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the thienyl ring, or
is pyrazolyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the pyrazole ring,
Rₚ³ are one to five identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy, C₁-C₆-alkylcarbonyl;
n is 0-5;
p is, depending on the number of free valencies, 0-4.

2. A benzamidoxime of the formula I as claimed in claim 1 where A is phenyl.

3. A benzamidoxime of the formula I as claimed in claim 1 where A is pyridyl.

4. A benzamidoxime of the formula I as claimed in claim 1 or 2 where Y is a carbon.

5. A benzamidoxime of the formula I as claimed in any of claims 1 - 3 where Rₙ¹ are one to five identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy.

6. A benzamidoxime of the formula I as claimed in any of claims 1 - 4 where
R² is phenyl-C₁-C₆-alkyl, which may carry one or more substituents selected from the group consisting of halogen, C₁-C₄-alkyl, C₁-C₄-haloalkyl, C₁-C₄-alkoxy and C₁-C₄-haloalkoxy on the phenyl ring, or
is thienyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the thienyl ring, or
is pyrazolyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the pyrazole ring.

7. A benzamidoxime of the formula I as claimed in any of claims 1 - 5 where Rₚ³ are one or two identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy.

8. A benzamidoxime of the formula I as claimed in claim 7 where Rₚ³ are hydrogen or C₁-C₄-alkyl.

9. A benzamidoxime of the formula I as claimed in claim 1 where:
A is an aryl or hetaryl radical from the group consisting of phenyl, pyridyl and thienyl;
Y is a carbon;
Rₙ¹ are one to five identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy;
R² is phenyl-C₁-C₆-alkyl, which may carry one or more substituents selected from the group consisting of halogen, C₁-C₄-alkyl, C₁-C₄-haloalkyl, C₁-C₄-alkoxy and C₁-C₄-haloalkoxy on the phenyl ring, or
is thienyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the thienyl ring, or
is pyrazolyl-C₁-C₄-alkyl, which may carry one or more substituents selected from the group consisting of halogen, C₁-C₄-alkyl, C₁-C₄-haloalkyl, C₁-C₄-alkoxy and C₁-C₄-haloalkoxy on the pyrazole ring,
Rₚ³ are one or two identical or different radicals from the group consisting of: hydrogen, halogen, C₁-C₆-alkyl, C₁-C₆-alkoxy, C₁-C₄-haloalkyl, C₁-C₄-haloalkoxy, C₁-C₄-alkylthio, C₁-C₄-alkoxyalkoxy;
n is 0-5;
p is 0-2.

10. The use of an amidoxime of the formula III 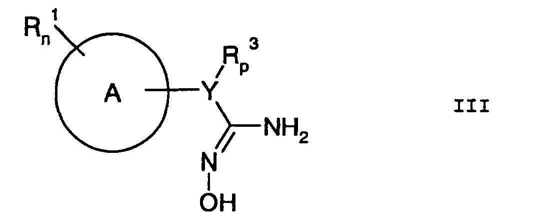 where Rₙ¹ and Rₚ³ are as defined in claim 1 for preparing amidoxime derivatives of the formula I.

11. An amidoxime derivative of the formula IV 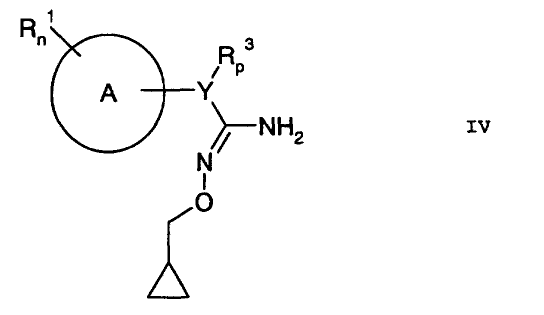 where Rₙ¹, Rₚ³, A and Y are as defined in claim 1.

12. The use of compounds of the formula IV as claimed in claim 11 for preparing benzamidoxime derivatives of the formula I.

13. The use of the benzamidoxime derivatives of the formula I as claimed in claims 1 - 9 for controlling harmful fungi.

14. A process for preparing the benzamidoxime derivatives of the formula I as claimed in any of claims 1 - 9, which comprises reacting benzonitriles of the formula II 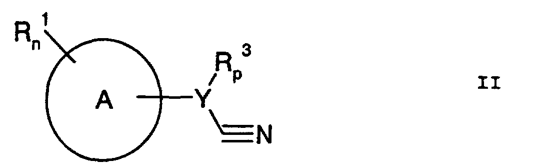 with hydroxylamine or salts thereof in aqueous solution, preferably at a pH greater than 8, to give benzamidoximes of the formula III 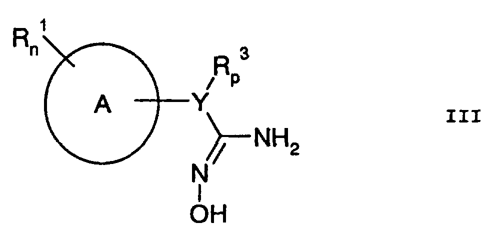 which are then alkylated using a cyclopropylmethyl halide to give benzamidoximes of the formula IV 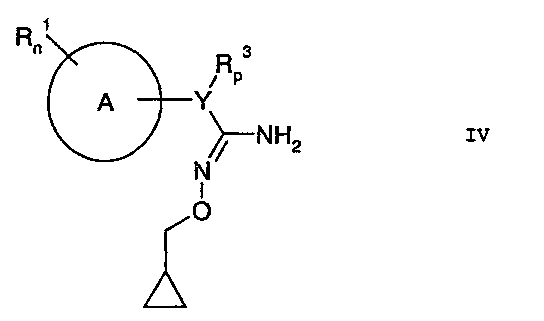 which are subsequently converted, using an appropriate acyl halide, into benzamidoxime derivatives of the formula I.

15. An agrochemical composition, comprising a fungicidally effective amount of at least one benzamidoxime derivative of the formula I as claimed in claims 1 - 9 and, if appropriate, agriculturally utilizable auxiliaries or additives.

16. A method for controlling harmful fungi, which comprises treating the harmful fungi, their habitat or the plants, areas, materials or spaces to be kept free from them with a fungicidally effective amount of a compound of the formula I or a fungicidal composition comprising a benzamidoxime derivative of the formula I as claimed in claim 15.

## Revendications

1. Dérivés de benzylamidoximes de formule I 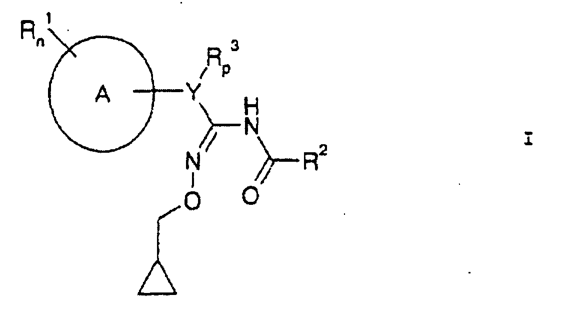 dans laquelle les radicaux ont la signification suivante :
A représente un radical aryle ou hétéroaryle du groupe constitué d'un radical phényle, d'un radical pyridyle ou d'un radical thiényle,
Y représente un groupe alkylène en C₁ à C₄ linéaire ou ramifié, un atome de carbone pouvant être remplacé par un atome d'oxygène, un atome d'azote ou un atome de soufre ou par un groupe cyclopropyle,
Rₙ¹ représente un à cinq radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy,
R² représente un groupe phényl(alkyle en C₁ à C₆), qui peut porter sur le cycle phényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄, ou
un groupe thiényl(alkyle en C₁ à C₄), qui peut porter sur le cycle thiényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄, ou
un groupe pyrazolyl(alkyle en C₁ à C₄), qui peut porter sur le cycle pyrazole un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄,
Rₚ³ représente un à cinq radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy, d'un groupe (alkyle en C₁ à C₆)carbonyle,
n vaut 0 à 5,
p vaut, en fonction des valences libres, 0 à 4.

2. Benzylamidoximes de formule I selon la revendication 1, dans laquelle A signifie un groupe phényle.

3. Benzylamidoximes de formule I selon l'une quelconque des revendications 1 à 8, dans laquelle A signifie un groupe pyridyle.

4. Benzylamidoximes de formule I selon la revendication 1 ou 2, dans laquelle Y représente un atome de carbone.

5. Benzylamidoximes de formule 1 selon l'une quelconque des revendications 1 à 3, dans laquelle Rₙ¹ signifie un à cinq radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy.

6. Benzylamidoximes de formule I selon l'une quelconque des revendications 1 à 4, dans laquelle
R² signifie un groupe phényl(alkyle en C₁ à C₆), qui peut porter sur le cycle phényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄, ou
un groupe thiényl(alkyle en C₁ à C₄), qui peut porter sur le cycle thiényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄, ou d'un groupe halogénoalcoxy en C₁ à C₄ ou
un groupe pyrazolyl(alkyle en C₁ à C₄), qui peut porter sur le cycle pyrazole un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄, ou d'un groupe halogénoalcoxy en C₁ à C₄.

7. Benzylamidoximes de formule I selon l'une quelconque des revendications 1 à 5, dans laquelle Rₚ³ signifie un à deux radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy.

8. Benzylamidoximes de formule I selon la revendication 7, dans laquelle Rₚ³ signifie un atome d'hydrogène ou un groupe alkyle en C₁ à C₄.

9. Benzylamidoximes de formule I selon la revendication 1, dans laquelle les radicaux ont la signification suivante :
A représente un radical aryle ou hétéroaryle du groupe constitué d'un radical phényle, d'un radical pyridyle ou d'un radical thiényle,
Y représente un atome de carbone,
Rₙ¹ représente un à cinq radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy,
R² représente un groupe phényl(alkyle en C₁ à C₆), qui peut porter sur le cycle phényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄ ou
un groupe thiényl(alkyle en C₁ à C₄), qui peut porter sur le cycle thiényle un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄, ou
un groupe pyrazolyl(alkyle en C₁ à C₄), qui peut porter sur le cycle pyrazole un ou plusieurs substituants choisis dans le groupe constitué d'un atome d'halogène, d'un groupe alkyle en C₁ à C₄, d'un groupe halogénoalkyle en C₁ à C₄, d'un groupe alcoxy en C₁ à C₄ ou d'un groupe halogénoalcoxy en C₁ à C₄,
Rₚ³ représente un à deux radicaux identiques ou différents du groupe constitué d'un atome d'hydrogène, d'un atome d'halogène, d'un groupe alkyle en C₁ à C₆, d'un groupe alcoxy en C₁ à C₆, d'un groupe halogénoalkyle en C₁ à C₄, d'un groupe halogénoalcoxy en C₁ à C₄, d'un groupe (alkyle en C₁ à C₄)thio, d'un groupe (alcoxy en C₁ à C₄)alcoxy,
n vaut 0 à 5.
p vaut 0 à 2.

10. Utilisation d'amidoximes de formule III 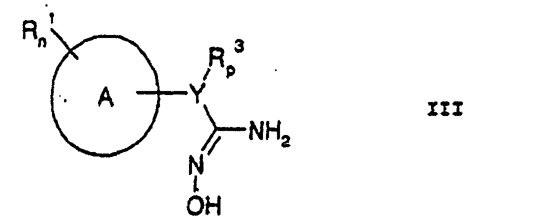 dans laquelle Rₙ¹ et Rₚ³ ont les significations indiquées dans la revendication 1 pour la préparation de dérivés d'amidoxime de formule I.

11. Dérivés d'amidoxime de formule IV 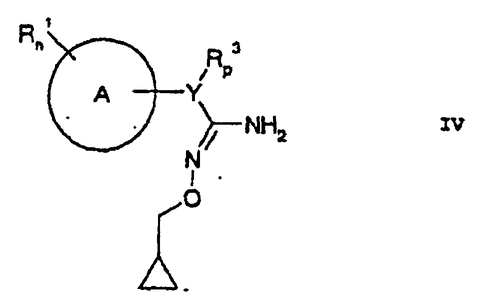 dans laquelle Rₙ¹, Rₚ³ A et Y ont les significations indiquées dans la revendication 1.

12. Utilisation de composés de formule IV selon la revendication 11 pour la préparation de dérivés de benzamidoxime de formule I.

13. Utilisation des dérivés de benzamidoxime de formule I selon les revendications 1 à 9 pour lutter contre les champignons nuisibles.

14. Procédé pour la préparation de dérivés de benzamidoxime de formule I selon l'une quelconque des revendications 1 à 9, **caractérisé en ce qu'**on transforme des benzonitriles de formule II 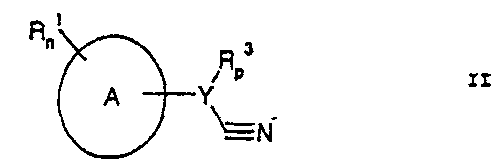 avec de l'hydroxylamine ou ses sels en solution aqueuse, de préférence à un pH supérieur à 8, en benzamidoximes de formule III 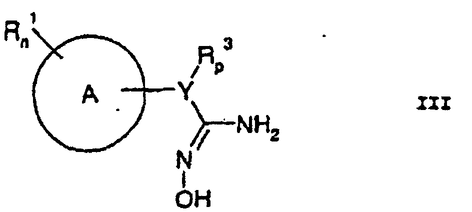 on alkyle ensuite ceux-ci avec un halogénure de cyclopropylméthyle en benzamidoximes de formule IV 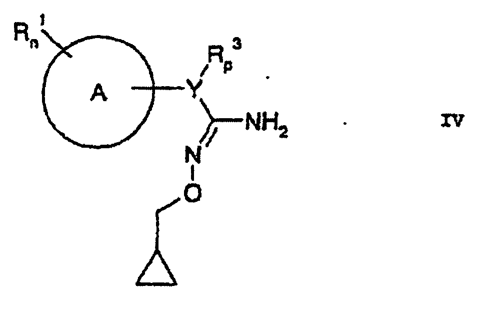 et on les transforme ensuite avec un halogénure d'acide correspondant en dérivés de benzamidoxime de formule I.

15. Composition agrochimique contenant une quantité efficace en tant que fongicide d'au moins un dérivé de benzamidoxime de formule 1 selon les revendications 1 à 9 ainsi que le cas échéant des adjuvants ou des additifs utilisables en agriculture.

16. Procédé pour lutter contre des champignons nuisibles, **caractérisé en ce qu'**on traite les champignons nuisibles, leur milieu ou les plantes, les surfaces, les matériaux ou les espaces à protéger contre eux avec une quantité efficace en tant que fongicide d'un composé de formule générale I ou un agent fongicide contenant un dérivé de benzamidoxime de formule I selon la revendication 16.
